# EUROPEAN PATENT SPECIFICATION

(11) **EP 1 462 803 B1**
(45) Date of publication and mention of the grant of the patent: **04.03.2009**
(21) Application number: 04006931.2
(22) Date of filing: 23.03.2004
(51) Int. Cl.: G01N 33/543

(54) **Biochemical analysis unit**
Biochemische Analysevorrichtung
Appareil d'analyse biochimique

(30) Priority: 25.03.2003 JP 2003082890
(43) Date of publication of application: 29.09.2004
(73) Proprietor: FUJIFILM Corporation, Minato-ku Tokyo (JP)
(72) Inventor: Kuruma, Koji, Minamiashigara-shi Kanagawa-ken (JP); Nakajima, Kenji, Minamiashigara-shi Kanagawa-ken (JP)
(74) Representative: Klunker . Schmitt-Nilson . Hirsch

(56) References cited:
- EP-A- 1 331 485
- US-A1- 2002 061 534

## Description

### BACKGROUND OF THE INVENTION

### Field of the Invention

This invention relates to a biochemical analysis unit according to the preamble of claim 1 and claim 6. It is used in an operation for detecting a receptor or a ligand by the utilization of a labeling substance.

### Description of the Related Art

Various micro array analysis systems and various macro array analysis systems have heretofore been used. With the micro array analysis systems and the macro array analysis systems, liquids containing ligands or receptors (i.e., the substances, which are capable of specifically binding to organism-originating substances and whose base sequences, base lengths, compositions, characteristics, and the like, are known) are spotted onto different positions on a surface of abiochemical analysis unit, such as amembrane filter, and a plurality of adsorptive regions are thereby formed on the surface of the biochemical analysis unit. Examples of the ligands or the receptors include hormones, tumor markers, enzymes, antibodies, antigens, abzymes, other proteins, nucleic acids, cDNA's, DNA's, and RNA's. Thereafter, a labeled receptor or a labeled ligand, which has been labeled with a radioactive labeling substance, a fluorescent labeling substance, a labeling substance capable of causing a chemical luminescence substrate to produce chemical luminescence when being brought into contact with the chemical luminescence substrate, or the like, is subjected to hybridization, or the like, with the ligands or the receptors, which are contained in the adsorptive regions of the biochemical analysis unit. The labeled receptor or the labeled ligand is thus specifically bound to at least one of the ligands or the receptors, which are contained in the adsorptive regions of the biochemical analysis unit. The labeled receptor or the labeled ligand is the substance, which has been sampled from an organism through extraction, isolation, or the like, or has been subjected to chemical treatment after being sampled, and which has been labeled with the radioactive labeling substance, the fluorescent labeling substance, the labeling substance capable of causing a chemical luminescence substrate to produce the chemical luminescence when being brought into contact with the chemical luminescence substrate, or the like. Examples of the labeled receptors or the labeled ligands include hormones, tumor markers, enzymes, antibodies, antigens, abzymes, other proteins, nucleic acids, DNA's, and mRNA's.

In cases where the labeled receptor or the labeled ligand has been labeled with the radioactive labeling substance, a stimulable phosphor layer of a stimulable phosphor sheet is then exposed to radiation radiated out from the radioactive labeling substance, which is contained selectively in the adsorptive regions of the biochemical analysis unit. Thereafter, the stimulable phosphor layer is exposed to stimulating rays, which cause the stimulable phosphor layer to emit light in proportion to the amount of energy stored on the stimulable phosphor layer during the exposure of the stimulable phosphor layer to the radiation. The light emitted by the stimulable phosphor layer is detected photoelectrically, and data for a biochemical analysis is thereby obtained.

In cases where the labeled receptor or the labeled ligand has been labeled with the fluorescent labeling substance, excitation light is irradiated to the adsorptive regions of the biochemical analysis unit, and the fluorescent labeling substance, which is contained selectively in the adsorptive regions of the biochemical analysis unit, is excited by the excitation light to produce fluorescence. The thus produced fluorescence is detected photoelectrically, and data for a biochemical analysis is thereby obtained.

In cases where the labeled receptor or the labeled ligand has been labeled with the labeling substance capable of causing a chemical luminescence substrate to produce the chemical luminescence when being brought into contact with the chemical luminescence substrate, the labeling substance, which is contained selectively in the adsorptive regions of the biochemical analysis unit, is brought into contact with the chemical luminescence substrate. Also, the chemicalluminescence produced bythelabelingsubstance is detected photoelectrically, and data for a biochemical analysis is thereby obtained.

The micro array analysis systems and the macro array analysis systems are described in, for example, U.S. Patent Laid-Open No. 20020061534.

With the micro array analysis systems and the macro array analysis systems described above, a large number of the adsorptive regions, to which the ligands or the receptors are bound, are capable of being formed at a high density at different positions on the surface of the biochemical analysis unit, and the labeled receptor or the labeled ligand, which has been labeled with the labeling substance, is capable of being subjected to the hybridization, or the like, with the ligands or the receptors, which have been bound to the adsorptive regions formed at a high density at different positions on the surface of the biochemical analysis unit. Therefore, the micro array analysis systems and the macro array analysis systems described above have the advantages in that a receptor or a ligand is capable of being analyzed quickly.

Heretofore, with the biochemical analysis systems using a biochemical analysis unit, the hybridization, or the like, has ordinarily been performed with a shaking technique. With the shaking technique, the biochemical analysis unit, on which the ligands or the receptors have been fixed, is put into a hybridization bag, and a reaction liquid, which contains the labeled receptor or the labeled ligand, is added into the hybridization bag. Also, vibrations are given to the hybridization bag, and the labeled receptor or the labeled ligand is thus moved through convection or diffusion within the hybridization bad. In this manner, the labeled receptor or the labeled ligand is specifically bound to at least one of the ligands or the receptors having been fixed on the biochemical analysis unit.

However, with the shaking technique described above, it is not always possible to achieve uniform contact of the reaction liquid containing the labeled receptor or the labeled ligand with the plurality of the adsorptive regions, which contain the ligands or the receptors. Therefore, the problems occur in that the ligands or the receptors and the labeled receptor or the labeled ligand cannot efficiently be subjected to the binding. In order to solve the problems described above, the applicant proposed a technique, wherein a reaction liquid containing a labeled receptor or a labeled ligand is forcibly caused to flow across each of adsorptive regions of a biochemical analysis unit, such that the labeled receptor or the labeled ligand may penetrate sufficiently into the interior of each of the adsorptive regions of the biochemical analysis unit. The proposed technique is described in U. S. Patent Laid-Open No. 20030148543.

### [Patent Literature 1]

U.S. Patent Laid-Open No. 20020061534

With the aforesaid technique, wherein the reaction liquid containing the labeled receptor or the labeled ligand is forcibly caused to flow across each of adsorptive regions of the biochemical analysis unit, the labeled receptor or the labeled ligand is capable of being caused to penetrate sufficiently into the interior of each of the adsorptive regions of the biochemical analysis unit. Accordingly, the limit of detection of the labeled receptor or the labeled ligand is capable of being enhanced. Also, in cases where the amount of the labeled receptor or the labeled ligand is small, the labeled receptor or the labeled ligand is capable of being detected with a high signal-to-noise ratio.

However, in cases where the reaction liquid containing the labeled receptor or the labeled ligand is forcibly caused to flow across each of adsorptive regions of the biochemical analysis unit, the problems often occur, depending upon the kindof the labeled receptor or the labeled ligand contained in the reaction liquid and the state of the labeled receptor or the labeled ligand, in that the labeled receptor or the labeled ligand is agglomerated and adsorbed to the surface of each of the adsorptive regions of the biochemical analysis unit. Also, in the cases of chemical luminescence techniques, wherein a ligand or a receptor is detected by use of an enzyme-labeled antibody, the problems occur in that the enzyme-labeled antibody clogs the porous adsorptive regions of the biochemical analysis unit, and a background of the light emission quantity obtained from the adsorptive regions becomes markedly high. The labeled receptor or the labeled ligand, which has thus been agglomerated, or the enzyme-labeled antibody, which has thus clogged the porous adsorptive regions of the biochemical analysis unit, causes a signal, which should not be detected, to occur and obstructs the formation of accurate data for a biochemical analysis.

Also, in cases where the porous adsorptive material, which constitutes each of the adsorptive regions of a biochemical analysis unit, is connected with the porous adsorptive material, which constitutes an adjacent adsorptive region, at one of surfaces of a base plate of the biochemical analysis unit, the problems occur in that the signal coming from the receptor or the ligand, which is to be detected from a certain hole of the base plate, passes through the porous adsorptive material, which constitutes each of the adsorptive regions and is connected with the porous adsorptive material constituting an adjacent adsorptive region at the one surface of the base plate, and the signal thus propagates toward an adjacent hole of the base plate. Therefore, in such cases, the signal detection cannot be performed such that only the signal, which comes from the receptor or the ligand that is to be detected from the certain hole described above, is capable of being detected. In such cases, accurate data for a biochemical analysis cannot be formed.

### SUMMARY OF THE INVENTION

The primary object of the present invention is to provide a biochemical analysis unit, which enables accurate detection of a signal coming from a labeling substance in an operation for detecting a receptor or a ligand by the utilization of a labeling substance.

Another object of the present invention is to provide abiochemical analysis unit, which enables detection of only a signal coming from a receptor or a ligand that is to be detected from a certain hole of a base plate of the biochemical analysis unit, and which thus enables accurately detection of the signal coming from each of the holes of the base plate.

The present invention provides a biochemical analysis unit, comprising the features of claim 1 or claim 6.

In the biochemical analysis unit in accordance with the present invention, the signal absorbing layer for absorbing the signal, which passes through the porous adsorptive material that is connected at the one surface of the base plate, and which thus propagates from a certain hole of the base plate toward an adjacent hole of the base plate, maybe formed at only an area of a continuous region of the porous adsorptive material that is connected at the one surface of the base plate, which area is located just under the base plate and is other than the areas of the adsorptive regions formed in the holes of the base plate. Alternatively, the signal absorbing layer may be formed at only an area, which is located just under each of the adsorptive regions formed in the holes of the base plate. As another alternative, the signal absorbing layer may be formed over an entire area of a continuous region of the porous adsorptive material that is connected at the one surface of the base plate.

The biochemical analysis unit comprises the base plate, which has the plurality of the holes, and the porous adsorptive material, which is filled in each of the plurality of the holes of the base plate and forms each of the plurality of the adsorptive regions. Also, each of the adsorptive regions is provided with the signal absorbing layer for absorbing the noise signal, which will otherwise be detected from the adsorptive region. The signal absorbing layer absorbs the noise signal, which comes from a labeled receptor or a labeled ligand having been agglomerated or an enzyme-labeled antibody having clogged the porous adsorptive regions of the biochemical analysis unit, and which should not be detected. Therefore, with the biochemical analysis unit in accordance with the present invention, accurate data for a biochemical analysis is capable of being obtained.

The biochemical analysis unit comprises the base plate, which has the plurality of the holes, and the porous adsorptive material, which is filled in each of the plurality of the holes of the base plate and forms each of the plurality of the adsorptive regions. Also, the porous adsorptive material, which constitutes each of the adsorptive regions, is connected with the porous adsorptive material, which constitutes an adjacent adsorptive region, at one of surfaces of the base plate. The biochemical analysis unit in accordance with the present invention further comprises the signal absorbing layer for absorbing the signal, which passes through the porous adsorptive material that is connected at the one surface of the base plate, and which thus propagates from a certain hole of the base plate toward an adjacent hole of the base plate. Therefore, with the biochemical analysis unit in accordance with the present invention, the problems are capable of being efficiently prevented from occurring in that the signal, which is to be detected from a certain hole of the base plate, passes through the porous adsorptive material, which is connected at the one surface of the base plate, and the signal thus propagates toward an adjacent hole of the base plate. Accordingly, only the signal, which comes from a receptor or a ligand that is to be detected from the certain hole described above, is capable of being detected. As a result, the signal coming from each of the holes of the base plate is capable of being detected accurately.

### BRIEF DESCRIPTION OF THE DRAWINGS

Figure 1 is a schematic perspective view showing a first embodiment of the biochemical analysis unit in accordance with the present invention,
Figure 2 is a schematic sectional view showing a part of the first embodiment of the biochemical analysis unit in accordance with the present invention,
Figure 3 is a schematic perspective view showing a second embodiment of the biochemical analysis unit in accordance with the present invention,
Figures 4A and 4B are schematic views showing an example of how the biochemical analysis unit in accordance with the present invention is produced,
Figure 5 is a schematic view showing a different example of how the biochemical analysis unit in accordance with the present invention is produced, and
Figure 6 is a schematic sectional view showing an example of a reactor, inwhich a reaction liquid is forcibly caused to flow.

### DESCRIPTION OF THE PREFERRED EMBODIMENTS

The present invention will hereinbelow be described in further detail with reference to the accompanying drawings.

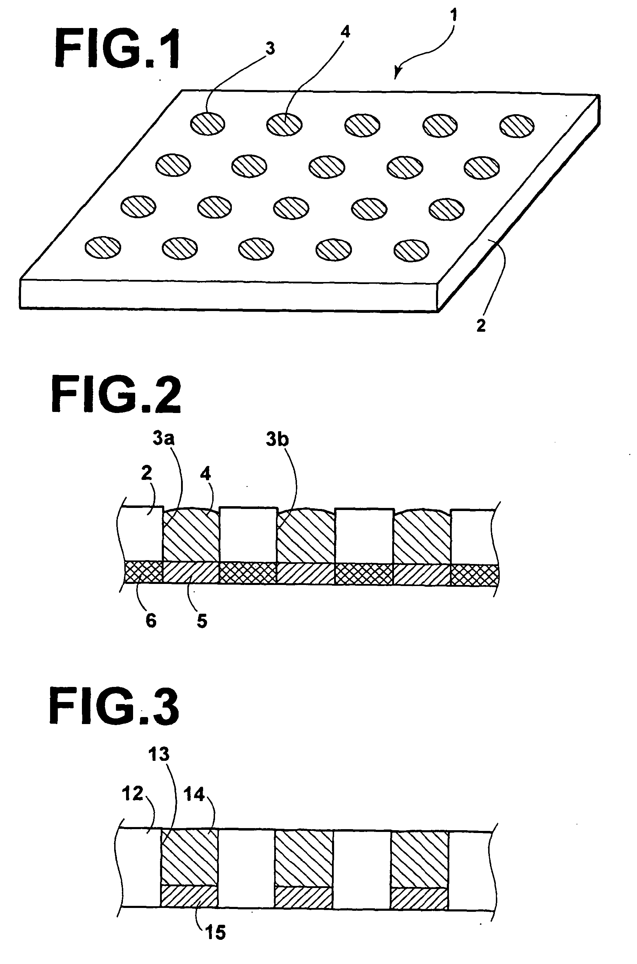

Figure 1 is a schematic perspective view showing a first embodiment of the biochemical analysis unit in accordance with the present invention. With reference to Figure 1, a biochemical analysis unit 1 comprises a base plate 2, which is provided with a plurality of holes 3, 3, ..., and a plurality of adsorptive regions 4, 4, ..., each of which is filled in one of the holes 3, 3, ... and comprises a porous adsorptive material adhered to the base plate 2.

Figure 2 is a schematic sectional view showing a part of the first embodiment of the biochemical analysis unit in accordance with the present invention. As illustrated in Figure 2, the porous adsorptive material, which constitutes each of the adsorptive regions 4, 4, ..., is connected with the porous adsorptive material, which constitutes an adjacent adsorptive region 4, at one of surfaces of the base plate 2. An area 5, at which the porous adsorptive material is connected at the one surface of the base plate 2, is provided with a signal absorbing layer 5 for absorbing a signal, which propagates from a certain hole 3a of the base plate 2 toward an adjacent hole 3b of the base plate 2. An underside area 6 located under the baseplate 2 is constitutedof the porous adsorptive material and the signal absorbing layer 5, which have been compressed by being pressed together against the bottom surface of the base plate 2.

A liquid containing a ligand or a receptor, which is to be fixed to each of the adsorptive regions of the biochemical analysis unit, is spotted from one side of each of the adsorptive regions onto each of the adsorptive regions. Therefore, more of the ligand or the receptor is fixed to the spotting side of each of the adsorptive regions . However, the liquid containing the ligand or the receptor infiltrates into each of the adsorptive regions and is also fixed to an area of each of the adsorptive regions, which area is lower than the middle area of each of the adsorptive regions. Therefore, in cases where a receptor or a ligand, which has been bound to the ligand or the receptor having been fixed to each of the adsorptive regions, is to be detected by the utilization of a labeling substance, the signal coming from the labeling substance is produced from an approximately entire area of each of the adsorptive regions. Accordingly, in the cases of the biochemical analysis unit 1, wherein the porous adsorptive material, which constitutes each of the adsorptive regions 4, 4, ..., is connected with the porous adsorptive material, which constitutes an adj acent adsorptive region 4, at the underside area 6 located under the base plate 2, the signal will pass through the underside area 6 and will thus propagate from the hole 3a toward the adjacent hole 3b. However, the biochemical analysis unit 1 in accordance with the present invention is provided with the signal absorbing layer 5 for absorbing the signal, which passes through the porous adsorptive material that is connected at the area under the base plate 2, and which thus propagates from the hole 3a toward the adjacent hole 3b of the base plate 2. Therefore, the effect of the signal propagating from the hole 3a toward the adj acent hole 3b is capable of being suppressed.

In the embodiment of Figure 2, the signal absorbing layer 5 is formed over an entire area of the continuous region of the porous adsorptive material that is connected at the one surface of the base plate 2. Alternatively, the signal absorbing layer may be formed at only the area, which is located just under each of the adsorptive regions 4, 4, ..., so as to close each of the holes 3a, 3b, ..., such that the signal absorbing layer is capable of absorbing the signal propagating from the hole 3a toward the adj acent hole 3b. As another alternative, the signal absorbing layer may be formed at only the area of the continuous region of the porous adsorptive material that is connected at the one surface of the base plate 2, which area is located just under the base plate 2 and is other than the areas of the adsorptive regions 4, 4, ... formed in the holes 3a, 3b, ... of the base plate 2.

However, in cases where the signal absorbing layer 5 is formed over the entire area of the continuous region of the porous adsorptive material that is connected at the one surface of the base plate 2, or in cases where the signal absorbing layer is formed at only the area, which is located just under each of the adsorptive regions 4, 4, ..., so as to close each of the holes 3a, 3b, ..., the signal absorbing layer is capable of absorbing the noise signal, which comes from a labeled receptor or a labeled ligand having been agglomerated and clinging to the signal absorbing layer located just under each of the adsorptive regions 4, 4, ..., an enzyme-labeled antibody having clogged the signal absorbing layer located just under each of the adsorptive regions 4, 4, ..., and the like, and which should not be detected. Therefore, in such cases, accurate data for a biochemical analysis is capable of being obtained.

Figure 3 is a schematic perspective view showing a second embodiment of the biochemical analysis unit in accordance with the present invention. In the second embodiment of the biochemical analysis unit in accordance with the present invention, the porous adsorptive material constituting the adsorptive regions is not connected at the one surface of the base plate. Specifically, the second embodiment of the biochemical analysis unit in accordance with the present invention comprises a base plate 12 having a plurality of holes 13, 13, ... The second embodiment of the biochemical analysis unit in accordance with the present invention also comprises adsorptive regions 14, 14, ..., each of which is constituted of the porous adsorptive material filled in one of the holes 13, 13, ... Each of the adsorptive regions 14, 14, ... is provided with a signal absorbing layer 15 for absorbing a noise signal, which will otherwise be detected from the adsorptive region 14.

Ordinarily, in cases where a reaction liquid containing a labeled receptor or the labeled ligand is forcibly caused to flow across each of the adsorptive regions of the biochemical analysis unit within a reactor during an operation for subjecting the labeled receptor or the labeled ligand to specific binding with the ligand or the receptor, which has been fixed to each of the adsorptive regions of the biochemical analysis unit, the problems occur in that the labeled receptor or the labeled ligand contained in the reaction liquid is apt to be agglomerated, and the labeled receptor or the labeled ligand having been agglomerated is apt to be adsorbed to the surface of each of the adsorptive regions, which surface is located on the upstream side with respect to the direction of the flow of the reaction liquid. Also, in the cases of a chemical luminescence technique, wherein a ligand or a receptor is detected by the utilization of a reaction of an enzyme-labeled antibody for decomposing a chemical luminescence substrate, the problems occur in that the enzyme-labeled antibody clogs the area of each of the adsorptive regions, which area is located on the upstream side with respect to the direction of the flow of a reaction liquid containing the enzyme-labeled antibody. In such cases, the problems occur in that a background of the light emission quantity obtained from the adsorptive regions becomes markedly high.

However, with the second embodiment of the biochemical analysis unit in accordance with the present invention, the signal absorbing layer 15 is formed on one side of each of the adsorptive regions 14, 14, ... of the biochemical analysis unit. Therefore, in cases where the biochemical analysis unit is set in the reactor in which the reaction liquid is to be forcibly caused to flow across each of the adsorptive regions 14, 14, ... of the biochemical analysis unit, the biochemical analysis unit may be set in an orientation such that the signal absorbing layer 15 stands facing the upstream side with respect to the direction of the flow of the reaction liquid. In such cases, the aforesaid labeled receptor or the labeled ligand having been agglomerated or the aforesaid clogging enzyme-labeled antibody clings to the signal absorbing layer 15. Therefore, at the time of signal detection, the signal detection may be performed from the side of each of the adsorptive regions 14, 14, ..., which side is opposite to the side provided with the signal absorbing layer 15. In such cases, the noise signal, which is produced by the aforesaid labeled receptor or the labeled ligand having been agglomerated or the aforesaid clogging enzyme-labeled antibody, is absorbed by the signal absorbing layer 15 and therefore is not detected. Accordingly, the data for biochemical analysis free from the noise signal is capable of being obtained.

In the embodiment of Figure 3, in each of the holes 13, 13, ... of the biochemical analysis unit, the signal absorbing layer 15 is located on one side of the hole 13, and the adsorptive region 14 is located on the other side of the hole 13. Alternatively, in each of the holes 13, 13, ... of the biochemical analysis unit, two adsorptive subregions constituting the adsorptive region 14 may be located so as to sandwich the signal absorbing layer 15.

Such that light scattering may be prevented from occurring within the biochemical analysis unit, the base plate 2 or the base plate 12 of the biochemical analysis unit should preferably be made from a material, which does not transmit radiation or light, or which attenuates radiation or light. The material for the formation of the base plate 2 or the base plate 12 should preferably be a metal or a ceramic material. Also, in cases where a plastic material, for which the hole making processing is capable of being performed easily, is employed as the material for the formation of the base plate 2 or the base plate 12, particles should preferably be dispersed within the plastic material, such that radiation or light is capable of being attenuated even further.

Examples of the metals, which may be utilized preferably for the formation of the base plate 2 or the base plate 12, include copper, silver, gold, zinc, lead, aluminum, titanium, tin, chromium, iron, nickel, cobalt, tantalum, and alloys, such as stainless steel and bronze. Examples of the ceramic materials, which may be utilized preferably for the formation of the base plate 2 or the base plate 12, include alumina, zirconia, magnesia, and quartz. Examples of the plastic materials, which may be utilized preferably for the formation of the base plate 2 or the base plate 12, include polyolefins, such as a polyethylene and a polypropylene; polystyrenes; acrylic resins, such as a polymethyl methacrylate; polyvinyl chlorides; polyvinylidene chlorides; polyvinylidene fluorides; polytetrafluoroethylenes; polychlorotrifluoroethylenes; polycarbonates; polyesters, such as a polyethylene naphthalate and a polyethylene terephthalate; aliphatic polyamides, such as a 6-nylon and a 6,6-nylon; polyimides; polysulfones; polyphenylene sulfides; silicon resins, such as a polydiphenyl siloxane; phenolic resins, such as novolak; epoxy resins; polyurethanes; celluloses, such as cellulose acetate and nitrocellulose; copolymers, such as a butadiene-styrene copolymer; and blends of plastic materials.

Also, in cases where the receptor or the ligand, which is to be detected, is subjected to the specific binding with the ligands or the receptors, each of which has been bound to one of the adsorptive regions 4, 4, ... or one of the adsorptive regions 14, 14, ..., and the receptor or the ligand, which has thus been bound to at least one of the ligands or the receptors having been bound to the adsorptive regions 4, 4, ... or the adsorptive regions 14, 14, ..., is to be detected by the utilization of the labeling substance, it is desired that the radiation or the light radiated out from the labeling substance within a hole 3 of the base plate 2 or a hole 13 of the base plate 12 be prevented from passing from the hole through the base plate wall to the adjacent hole. Therefore, in cases where the base plate 2 or the base plate 12 is constituted of a plastic material, in order for the radiation or the light to be attenuated, theplasticmaterial shouldpreferablybe loaded with particles of metal oxides, glass fibers, or the like. Examples of the metal oxides include silicon dioxide, alumina, titanium dioxide, iron oxide, and copper oxide. However, themetal oxides are not limited to those enumerated above.

The radiation attenuating properties or the light attenuating properties should preferably be such that, when the radiation or the light, which is radiated out from the labeling substance within the hole 3 or 13, has passed from the hole 3 or 13 through the base plate wall to the adjacent hole 3 or 13, the intensity of the radiation or the light reduces to an intensity of at most 1/5 of the original intensity. The radiation attenuating properties or the light attenuating properties should more preferably be such that the intensity of the radiation or the light having passed through the base plate wall in the manner described above reduces to an intensity of at most 1/10 of the original intensity.

In order for the radiation, such as electron rays, coming from a radioactive labeling substance to be blocked efficiently, the mean density of the base plate 2 or the base plate 12 may ordinarily be at least 0.6g/cm³. The mean density of the base plate 2 or the base plate 12 should preferably fall within the range of 1g/cm³ to 20g/cm³, and should more preferably fall within the range of 2g/cm³ to 10g/cm³. Since the transmission distance of the electron rays is in inverse proportion to the density, in cases where the radioactive labeling substance is an ordinary radioactive isotope (RI), such as ³²P, ³³P, ³⁵S, or ¹⁴C, and the mean density of the base plate 2 or the base plate 12 falls within the range described above, the electron rays coming from the RI of the sample, which is fixed within each of the holes 3, 3, ... or each of the holes 13, 13, ..., is capable of being blocked by the partition wall of the base plate 2 or the base plate 12, and the problems are capable of being prevented from occurring in that resolution of a radiation image is adversely affected by transmission and scattering of the electron rays.

The thickness of the base plate 2 or the base plate 12 may ordinarily fall within the range of 50µm to 1,000µm, and should preferably fall within the range of 100µm to 500µm.

Such that the density of the holes 3, 3, ... made through the base plate 2 or the density of the holes 13, 13, ... made through the base plate 12 may be enhanced, the area (size) of the opening of each of the holes 3, 3, ... or each of the holes 13, 13, ... may ordinarily be smaller than 5mm². The area of the opening of each of the holes 3, 3, ... or each of the holes 13, 13, ... should preferably be smaller than 1mm², should more preferably be smaller than 0. 3mm², and should most preferably be smaller than 0.01mm². Also, the area of the opening of each of the holes 3, 3, ... or each of the holes 13, 13, ... should preferably be at least 0.001mm².

The pitch of the holes 3, 3, ... or the holes 13, 13, ... (i.e., the distance between the center points of two holes which are adjacent to each other) should preferably fall within the range of 0.05mm to 3mm. Also, the spacing between two adjacent holes 3, 3 or between two adjacent holes 13, 13 (i.e., the shortest distance between edges of two adjacent holes 3, 3 or two adjacent holes 13, 13) should preferably fall within the range of 0.01mm to 1.5mm. The number (the array density) of the holes 3, 3, ... or the holes 13, 13, ... may ordinarilybe at least 10 holes/cm² . The number (the array density) of the holes 3, 3, ... or the holes 13, 13, ... should preferably be at least 100 holes/cm², shouldmore preferably be at least 500 holes/cm², and shouldmost preferablybe at least 1,000 holes/cm². Also, the number (the array density) of the holes 3, 3, ... or the holes 13, 13, ... should preferably be at most 100, 000 holes/cm², and should more preferably be at most 10,000 holes/cm². The holes 3, 3, ... or the holes 13, 13, ... need not necessarily be arrayed at equal spacing as illustrated in Figure 1. For example, the holes 3, 3, ... or the holes 13, 13, ... may be grouped into several number of blocks (units) comprising a plurality of holes and may be formed in units of the blocks.

Perforation of the plurality of the holes 3, 3, ... through the base plate 2 or the perforation of the plurality of the holes 13, 13, ... through the base plate 12 may be performed with, for example, a punching technique for punching with a pin, a technique for electrical discharge machining, in which a pulsed high voltage is applied across electrodes in order to volatilize the base plate material, an etching technique, or a laser beam irradiation technique. In cases where the material of the base plate is a metal material or a plastic material, the biochemical analysis unit may be prepared with an operation for performing corona discharge or plasma discharge on the surface of the base plate, applying an adhesive agent to the surface of the base plate, and laminating the porous material for the formation of the adsorptive regions by use of means, such as a press. The adhesive agent described above should preferably be styrene-butadiene rubber, acrylonitrile-butadiene rubber, or the like. The application of the adhesive agent should preferably be performed with a roll coating technique, a wire bar coating technique, a dip coating technique, a blade coating technique, or the like. Also, in cases where the porous material for the formation of the adsorptive regions is pressed against the base plate, the base plate and the porous material for the formation of the adsorptive regions may be divided previously into a plurality of sheets, and the plurality of the sheets may be pressed intermittently. Alternatively, a long web of the base plate and a long web of the porous material for the formation of the adsorptive regions may be conveyed continuously between two rolls.

In the biochemical analysis unit in accordance with the present invention, as the porous adsorptive material for the formation of the adsorptive regions of the biochemical analysis unit, a porous quality material or a fiber material may be utilized preferably. The porous quality material and the fiber material may be utilized in combination in order to form the adsorptive regions of the biochemical analysis unit. In the biochemical analysis unit in accordance with the present invention, the porous adsorptive material, which may be utilized for the formation of the adsorptive regions of the biochemical analysis unit, may be an organic material, an inorganic material, or an organic-inorganic composite material.

The organic porous quality material, which may be utilized for the formation of the adsorptive regions of the biochemical analysis unit, may be selected from a wide variety of materials. However, the organic porous quality material should preferably be a carbon porous quality material, such as active carbon, or a porous quality material capable of forming amembrane filter. As the porous quality material capable of forming a membrane filter, a polymer soluble in a solvent should preferably be utilized. Examples of the polymers soluble in a solvent include cellulose derivatives, such as nitrocellulose, regenerated cellulose, cellulose acetate, and cellulose acetate butyrate; aliphatic polyamides, such as a 6-nylon, a 6,6-nylon, and a 4,10-nylon; polyolefins, such as a polyethylene and a polypropylene; chlorine-containing polymers, such as a polyvinyl chloride and a polyvinylidene chloride; fluorine resins, suchasapolyvinylidenefluoride and a polytetrafluoride; polycarbonates; polysulfones; alginic acids and alginic acid derivatives, such as alginic acid, calcium alginate, and an alginic acid-polylysine polyion complex; and collagen. Copolymers or composite materials (mixture materials) of the above-enumerated polymers may also be utilized.

The fiber material, which may be utilized for the formation of the adsorptive regions of the biochemical analysis unit, may be selected from a wide variety of materials. Examples of the fiber materials, which may be utilized preferably, include the cellulose derivatives and the aliphatic polyamides enumerated above.

The inorganic porous quality material, which may be utilized for the formation of the adsorptive regions of the biochemical analysis unit, may be selected from a widevarietyofmaterials. Examples of the inorganic porous qualitymaterials, whichmaybeutilizedpreferably, include metals, such as platinum, gold, iron, silver, nickel, and aluminum; oxides of metals, and the like, such as alumina, silica, titania, and zeolite; metal salts, such as hydroxyapatite and calcium sulfate; and composite materials of the above-enumerated materials.

Also, the signal absorbing layer is capable of being formed with a process, wherein a substance capable of absorbing the signal, such as a light signal or a radiation signal, is mixed with the porous adsorptive material described above. In cases where the signal is a chemical luminescence signal or a fluorescence signal, the signal absorbing layer is capable of being formed with a process, wherein a dye, or the like, capable of absorbing light having wavelengths falling within the wavelength range of the chemical luminescence or the fluorescence is mixed with the porous adsorptive material described above. In cases where the signal is the radiation signal, the signal absorbing layer is capable of being formed with a process, wherein fine particles of a heavy metal, such as lead or tungsten, which acts as a radiation blocking substance, are mixed with the porous adsorptive material described above.

Each of the adsorptive regions and the signal absorbing layermaybe formed with aprocess describedbelow. Specifically, a solution (hereinbelow referred to as the dope) containing the adsorptive material for the formation of each of the adsorptive regions and a dope containing the adsorptive material for the formation of the signal absorbing layer are successively cast or coated on a support, and the resulting casting layers or the resulting coating layers are then dipped in bad solvents for the polymers of the porous films or in a mixed solvent of good solvents and bad solvents for the polymers and thereafter subjected to washing with water and drying. Alternatively, each of the adsorptive regions and the signal absorbing layer may be formed with a different process, wherein one of different kinds of dopes is cast or coated on a support, the other dope is cast or coated on a different support, and each of the resulting casting layers or each of the resulting coating layers is then dried little by little.

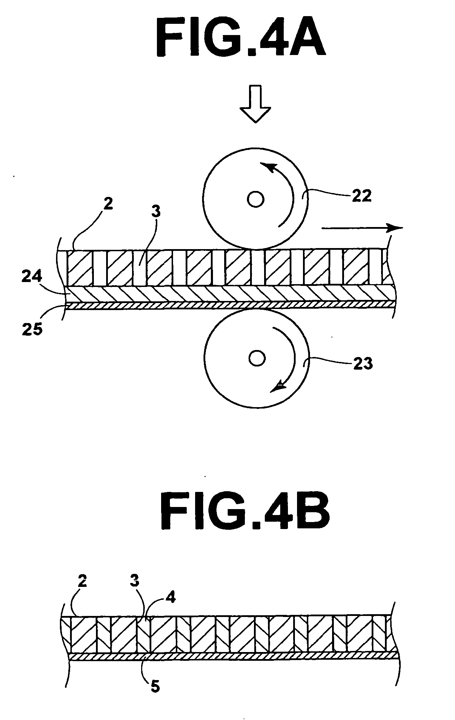

The aforesaid first embodiment of the biochemical analysis unit in accordance with the present invention, wherein the porous adsorptive material constituting the adsorptive regions is connected at the one surface of the base plate, may be produced with, for example, the process described below. Figures 4A and 4B are schematic views showing an example of how the first embodiment of the biochemical analysis unit in accordance with the present invention is produced. In the example shown in Figures 4A and 4B, the first embodiment of the biochemical analysis unit in accordance with the present invention is produced with a pressing technique, wherein a porous film 24 for constituting the adsorptive regions 4, 4, ..., a porous film 25 for constituting the signal absorbing layer 5, and the base plate 2 are superposed one upon another and pressed together, and the porous film 24 is thereby press-fitted into the holes 3, 3, ... of the base plate 2. With the pressing technique, the porous film 24 is capable of being press-fitted into the holes 3, 3, ... of the base plate 2 such that little change occurs with the pore diameters of the pores of the region of the porous film 24, which region is press-fitted into each of the holes 3, 3, ...

As illustrated in Figure 4A, the base plate 2 having the holes 3, 3, ..., the porous film 24, and the porous film 25 are superposed one upon another and pressed together by being passed between a press roll 22 and a back-up roll 23. In this manner, as illustrated in Figure 4B, the porous film 24 is capable of being press-fitted into the holes 3, 3, ... of the base plate 2. In such cases, the porous film 24 and the porous film 25 may be softened with a technique wherein, for example, the press roll 22 and the back-up roll 23 are heated.

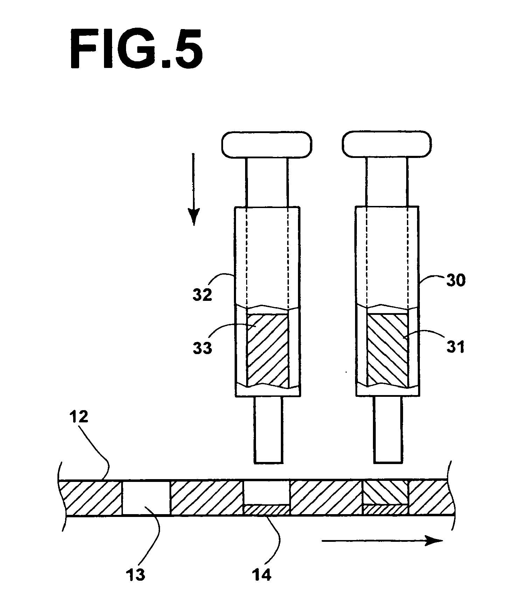

The aforesaid second embodiment of the biochemical analysis unit in accordance with the present invention, wherein the porous adsorptive material constituting the adsorptive regions is not connected at the one surface of the base plate, may be produced with, for example, the process described below. Figure 5 is a schematic view showing an example of how the second embodiment of the biochemical analysis unit in accordance with the present invention is produced. As illustrated in Figure 5, a dispenser 30 for injecting a dope 31 into the holes 13, 13, ... of the base plate 12 and a dispenser 32 for injecting a dope 33 into the holes 13, 13, ... of the base plate 12 are located above the base plate 12, which is conveyed continuously or intermittently. In Figure 5, as an aid in clarifying the relationship between each of the dopes 31 and 33 and the formation of the corresponding layer, the contents of each of the dopes 31 and 33 are shown by the illustration identical with the illustration of the corresponding layer. The dispenser 32 intermittently injects the dope 33 into each of the holes 13, 13, ... of the base plate 12. Thereafter, the dispenser 30 intermittently injects the dope 31 onto the dope 33 having been injected into each of the holes 13, 13, ... of the base plate 12. After the dopes 31 and 33 have been injected into each of the holes 13, 13, ... of the base plate 12, air having a controlled temperature and a controlled humidity is fed over the base plate 12 at a predetermined flow rate, and the solvents contained in the dopes 31 and 33 are vaporized little by little. In this manner, the biochemical analysis unit, wherein the adsorptive region 14 and the signal absorbing layer have been formed in each of the holes 13, 13, ... of the base plate 12, is capable of being produced.

The biochemical analysis unit in accordance with the present invention is applicable broadly to various assay processes for:
i) obtaining a biochemical analysis unit provided with a plurality of porous adsorptive regions, to which ligands or receptors have been bound respectively,
ii) subjecting a reaction liquid containing at least one kind of a receptor or at least one kind of a ligand to specific binding with the ligands or the receptors, each of which has been bound to one of the porous adsorptive regions of the biochemical analysis unit, the receptor or the ligand being thereby specifically bound to at least one of the ligands, each of which has been bound to one of the porous adsorptive regions of the biochemical analysis unit, or at least one of the receptors, each of which has been bound to one of the porous adsorptive regions of the biochemical analysis unit, and
iii) detecting the receptor or the ligand, which has thus been specifically bound to at least one of the ligands or at least one of the receptors, by the utilization of a labeling substance.

In a first aspect, the biochemical analysis unit in accordance with the present invention is applicable to an assay process for:
i) obtaining a biochemical analysis unit provided with a plurality of porous adsorptive regions, to which ligands or receptors have been bound respectively,
ii) subjecting a reaction liquid containing at least one kind of a labeled receptor or at least one kind of a labeled ligand, which has been labeled with a labeling substance, to specific binding with the ligands or the receptors, each of which has been bound to one of the porous adsorptive regions of the biochemical analysis unit, the labeled receptor or the labeled ligand being thereby specifically bound to at least one of the ligands, each of which has been bound to one of the porous adsorptive regions of the biochemical analysis unit, or at least one of the receptors, each of which has been bound to one of the.porous adsorptive regions of the biochemical analysis unit, and
iii) detecting the labeled receptor or the labeled ligand, which has thus been specifically bound to at least one of the ligands or at least one of the receptors.

In such cases, the labeled receptor or the labeled ligand is the substance, which has been sampled from an organism through extraction, isolation, or the like, or has been subj ected to chemical treatment after being sampled, and which has been labeled with the labeling substance. The labeled receptor or the labeled ligand is capable of undergoing the specific binding with at least one of the ligands, each of which has been bound to one of the porous adsorptive regions of the biochemical analysis unit, or at least one of the receptors, each of which has been bound to one of the porous adsorptive regions of the biochemical analysis unit. Examples of the labeled receptors or the labeled ligands include hormones, tumor markers, enzymes, antibodies, antigens, abzymes, other proteins, nucleic acids, DNA's, and mRNA's.

Examples of the labeling substances include a radioactive labeling substance, a fluorescent labeling substance, and a labeling substance capable of causing a chemical luminescence substrate to produce the chemical luminescence when being brought into contact with the chemical luminescence substrate. The labeling substance may be a substance, which is capable of producing radiation by itself, a substance, which is capable of emitting light by itself, a substance, which is capable of forming a color by itself, or a substance, which is capable of producing fluorescence by itself when being exposed to light. Alternatively, the labeling substance may be a substance, which is capable of causing a chemical substance to emit light, to forma color, or to produce the fluorescence through, for example, decomposition or reaction of the chemical substance when being brought into contact with the chemical substance. As for the former type of the labeling substance, a radioactive isotope may be employed as the radiation producing labeling substance. Also, an acridinium ester, or the like, may be employed as the light emitting labeling substance. Further, gold colloidal particles, or the like, may be employed as the color forming labeling substance. Furthermore, fluorescein, or the like, may be employed as the fluorescent labeling substance. As the latter type of the labeling substance, an enzyme maybe employed. Examples of the enzymes include alkaline phosphatase, peroxidase, luciferase, and β-galactosidase. When one of the above-enumerated enzymes acting as the labeling substance is brought into contact with a chemical luminescence substrate, a dye substrate, or a fluorescence substrate, the enzyme is capable of causing the chemical luminescence substrate to produce the chemical luminescence, causing the dye substrate to forma color, or causing the fluorescence substrate to produce the fluorescence.

By way of example, in cases where the enzyme is alkaline phosphatase, peroxidase, or luciferase, the chemical luminescence substrate may be dioxetane, luminol, or luciferin, respectively. In cases where the enzyme is alkaline phosphatase,the dyesubstrate maybe p-nitrophenyl phosphate. In cases where the enzyme is β-galactosidase, the dye substrate may be p-nitrophenyl-β-D-galactoside, or the like. In cases where the enzyme is alkaline phosphatase, the fluorescence substrate may be 4-methylumbelliferphosphoric acid. In cases where the enzyme is peroxidase, the fluorescence substrate may be 3-(4-hydroxyphenyl)-propionic acid. In cases where the enzyme is β-galactosidase, the fluorescence substrate may be 4-methylumbellifer-β-D-galactoside, or the like.

In a second aspect, the biochemical analysis unit in accordance with the present invention is applicable to an assay process for:
i) obtaining a biochemical analysis unit provided with a plurality of porous adsorptive regions, to which ligands or receptors have been bound respectively,
ii) subjecting a reaction liquid containing at least one kind of a receptor or at least one kind of a ligand to specific binding with the ligands or the receptors, each of which has been bound to one of the porous adsorptive regions of the biochemical analysis unit, the receptor or the ligand being thereby specifically bound to at least one of the ligands, each of which has been bound to one of the porous adsorptive regions of the biochemical analysis unit, or at least one of the receptors, each of which has been bound to one of the porous adsorptive regions of the biochemical analysis unit,
iii) subjecting a labeled body, which has been labeled with a labeling substance, to specific binding with the receptor or the ligand having been specifically bound to at least one of the ligands, each of which has been bound to one of the porous adsorptive regions of the biochemical analysis unit, or at least one of the receptors, each of which has been bound to one of the porous adsorptive regions of the biochemical analysis unit, and
iv) detecting the receptor or the ligand, which has been specifically bound to at least one of the ligands or at least one of the receptors.

The aforesaid second aspect of the assay process is the so-called sandwich technique, wherein the receptor or the ligand, which is to be detected, is sandwiched between the ligand or the receptor, which has been bound to the adsorptive region, and the labeled body. In this case, the receptor or the ligand, which is to be detected, is the substance, which has been sampled from an organism through extraction, isolation, or the like, or has been subjected to chemical treatment after being sampled, and which has been labeled with the labeling substance. The receptor or the ligand is capable of undergoing the specific binding with at least one of the ligands, each of which has been bound to one of the porous adsorptive regions of the biochemical analysis unit, or at least one of the receptors, each of which has been bound to one of the porous adsorptive regions of the biochemical analysis unit. Examples of the receptors or the ligands, which are to be detected, include hormones, tumor markers, enzymes, antibodies, antigens, abzymes, other proteins, nucleic acids, DNA's, andmRNA's.

The labeled body, which has been labeled with the labeling substance, is a body, which has been labeled with the labeling substance described above and is capable of undergoing the specific binding with a reaction site of the receptor or the ligand, which is to be detected. Examples of the labeledbodies include antigens, antibodies, hormones, tumor markers, enzymes, abzymes, other proteins, nucleic acids, cDNA's, DNA's, and RNA's, whose characteristics, compositions, structures, base sequences, base lengths, and the like, are known.

In a third aspect, the biochemical analysis unit in accordance with the present invention is applicable to an assay process for:
i) obtaining a biochemical analysis unit provided with a plurality of porous adsorptive regions, to which ligands or receptors have been bound respectively,
ii) subjecting a reaction liquid containing at least one kind of an auxiliary substance-bound receptor or at least one kind of an auxiliary substance-bound ligand, to which an auxiliary substance has been bound, to specific binding with the ligands or the receptors, each of which has been bound to one of the porous adsorptive regions of the biochemical analysis unit, the auxiliary substance-bound receptor or the auxiliary substance-bound ligand being thereby specifically bound to at least one of the ligands, each of which has been bound to one of the porous adsorptive regions of the biochemical analysis unit, or at least one of the receptors, each of which has been bound to one of the porous adsorptive regions of the biochemical analysis unit,
iii) subjecting an auxiliary substance-combinable labeling substance, which is capable of undergoing specificbindingwith the auxiliary substance, to specific binding with the auxiliary substance-bound receptor or the auxiliary substance-bound ligand having been specifically bound to at least one of the ligands, each of which has been bound to one of the porous adsorptive regions of the biochemical analysis unit, or at least one of the receptors, each of which has been bound to one of the porous adsorptive regions of the biochemical analysis unit, and
iv) detecting the auxiliary substance-bound receptor or the auxiliary substance-bound ligand, which has been specifically bound to at least one of the ligands or at least one of the receptors.

The auxiliary substance is a substance capable of undergoing the binding with the auxiliary substance-combinable labeling substance. Examples of preferable auxiliary substances include antigens, such as digoxigenin, biotin, avidin, and fluorescein, and antibodies with respect to the above-enumerated antigens. Also, the auxiliary substance may be a biological binding partner, such as avidin with respect to biotin. In this case, the auxiliary substance-combinable labeling substance is a substance, which is capable of undergoing the specific binding with the auxiliary substance and has been labeled with the labeling substance described above.

How a biochemical analysis using the biochemical analysis unit in accordance with the present invention is performedwill be describedhereinbelowby taking a chemical luminescence technique as an example.

In the chemical luminescence technique using the biochemical analysis unit in accordance with the present invention, firstly, the ligands or the receptors are bound respectively to the adsorptive regions of the biochemical analysis unit, which is provided with the plurality of the adsorptive regions. At this time, the ligands or the receptors are spotted from the side of the biochemical analysis unit, which side is opposite to the side provided with the signal absorbing layer. After the ligands or the receptors have thus been spotted respectively onto the adsorptive regions of the biochemical analysis unit, the ligands or the receptors are capable of being fixed to the adsorptive regions with ultraviolet light irradiation, or the like.

Thereafter, a labeled receptor or a labeled ligand, which has been labeled with a labeling substance, is subjected to specific binding with the ligands or the receptors, each of which has been bound to one of the adsorptive regions of the biochemical analysis unit. In order to perform the specific binding of labeled receptor or the labeled ligand with the ligands or the receptors, each of which has been bound to one of the adsorptive regions of the biochemical analysis unit, a reactor, in which a reaction liquid is capable of being forcibly caused to flow such that the reaction liquid flows across each of the adsorptive regions of the biochemical analysis unit, is utilized.

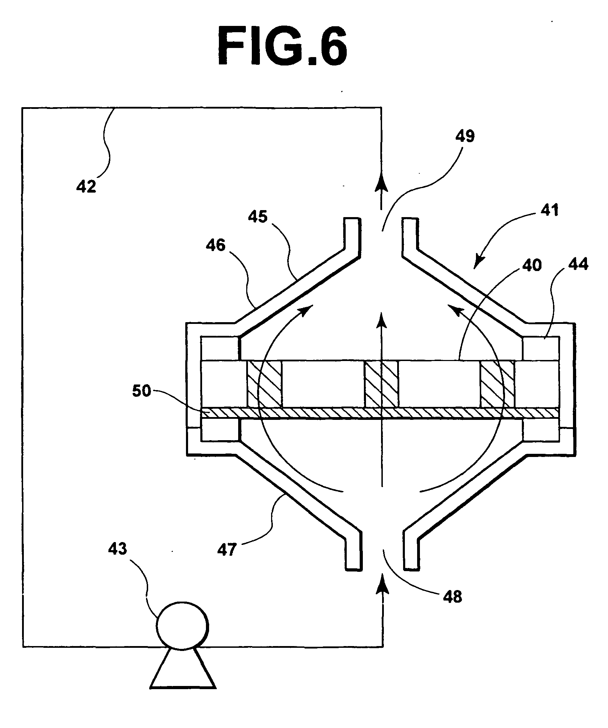

Figure 6 is a schematic sectional view showing an example of a reactor, in which a reaction liquid is capable of being forcibly caused to flow. With reference to Figure 6, the reactor comprises a reaction vessel 41, a liquid circulating pipe 42 and a pump 43. The reaction vessel 41 is provided with abiochemical analysis unit support section 44, which supports a biochemical analysis unit 40 and has sealing functions for preventing liquid leakage. A reaction vessel main body 45 of the reaction vessel 41 comprises a reaction vessel upper half 46 and a reaction vessel lower half 47. The reaction vessel upper half 46 is releasably secured to the reaction vessel main body 45. When the biochemical analysis unit 40 is to be set within the reaction vessel 41, the reaction vessel upper half 46 is dismounted from the reaction vessel main body 45, and the biochemical analysis unit 40 is set within the reaction vessel 41. A bottom wall of the reaction vessel lower half 47 is provided with a liquid inlet 48, through which a liquid is capable of flowing. Also, a top wall of the reaction vessel upper half 46 is provided with a liquid outlet 49, through which the liquid is capable of flowing. Further, the liquid circulating pipe 42 is releasably fitted to the liquid inlet 48 and the liquid outlet 49 of the reaction vessel 41. The reactor is constituted such that the liquid is introduced by the pump 43 into the reaction vessel main body 45 through the liquid inlet 48, passed through the biochemical analysis unit 40, discharged through the liquid outlet 49, and circulated through the liquid circulating pipe 42.

The biochemical analysis unit 40 provided with the adsorptive regions, to which the ligands or the receptors have been bound respectively, is set in the reactor. Also, the reaction liquid containing the labeled receptor or the labeled ligand is introduced into the reaction vessel 41. Thereafter, the pump 43 is actuated, and the reaction liquid is forcibly caused to flow such that the reaction liquid flows across each of the adsorptive regions of the biochemical analysis unit 40. In this manner, the labeled receptor or the labeled ligand is capable of being subjected to the specific binding with the ligands or the receptors, which have been bound respectively to the adsorptive regions of the biochemical analysis unit 40. The biochemical analysis unit 40 is set in the reactor in an orientation such that a signal absorbing layer 50 of the biochemical analysis unit 40 stands facing the upstream side with respect to the direction of the flow of the reaction liquid, which is forcibly caused to flow. Since the biochemical analysis unit 40 is set in the orientation described above, in cases where the labeled receptor or the labeled ligand contained in the reaction liquid is agglomerated, the labeled receptor or the labeled ligand having thus been agglomerated is adsorbed to the signal absorbing layer 50. Therefore, in cases where the signal coming from each of the adsorptive regions of the signal absorbing layer 50 is detected from the side, which is opposite to the side provided with the signal absorbing layer 50, the signal coming from the labeled receptor or the labeled ligand having been agglomerated is absorbed by the signal absorbing layer 50 and therefore is not detected. Accordingly, only the signal coming from the labeled receptor or the labeled ligand, which has been specifically bound to at least one of the ligands having been fixed to the adsorptive regions or at least one of the receptors having been fixed to the adsorptive regions, is capable of being detected.

In the aforesaid example, the specific binding is performed by use of the reactor, which is capable of forcibly causing the reaction liquid to flow such that the reaction liquid flows across each of the adsorptive regions of the biochemical analysis unit. However, the biochemical analysis unit in accordance with the present invention is not limited to the use within the reactor described above. For example, the biochemical analysis unit in accordance with the present invention may be utilized for the shaking technique, wherein the biochemical analysis unit and the reaction liquid are put into a hybridization bag, vibrations are given to the hybridization bag, and the labeled receptor or the labeled ligand is thus moved through convection or diffusion and is specifically bound to one of the ligands or the receptors having been fixed to the adsorptive regions of the biochemical analysis unit.

In order for the labeled receptor or the labeled ligand, which has not been specifically bound to the ligands or the receptors having been bound respectively to the porous adsorptive regions of the biochemical analysis unit, to be removed, the biochemical analysis unit having been set within the reaction vessel should preferably be washed with a technique for forcibly causing a washing liquid to flow across each of the adsorptive regions. In such cases, since the washing liquid is forcibly caused to flow across each of the adsorptive regions, the labeled receptor or the labeled ligand, which has not been specifically bound to the ligands or the receptors having been bound respectively to the porous adsorptive regions of the biochemical analysis unit, is capable of beingpeeledoff andremoved efficiently. Therefore, the washing efficiency is capable of being enhanced markedly.

After a reaction liquid, which contains an enzyme-labeled antibody described later, is forcibly caused to flow such that the reaction liquid flows across each of the adsorptive regions of the biochemical analysis unit, and the enzyme-labeled antibody is thus subjected to the specific binding with the labeled receptor or the labeled ligand, the enzyme-labeled antibody, which has not been specifically bound to the labeled receptor or the labeled ligand, may be removed. In cases where the enzyme-labeled antibody, which has not been specifically bound to the labeled receptor or the labeled ligand, is to be removed, the washing process described above should preferably be performed. In this manner, the enzyme-labeled antibody, which has not been specificallybound to the labeled receptor or the labeled ligand, is capable of being peeled off and removed efficiently. Therefore, the washing efficiency is capable of being enhanced markedly.

Before the enzyme-labeled antibody is subjected to the specific binding with the labeled receptor or the labeled ligand having been specifically bound to at least one of the ligands, each of which has been bound to one of the adsorptive regions of the biochemical analysis unit, or at least one of the receptors, each of which has been bound to one of the adsorptive regions of the biochemical analysis unit, the adsorptive regions should preferably be blocked with a blocking process, wherein a blocking buffer with respect to the enzyme-labeled antibody is forcibly caused to flow such that the blocking buffer flows across each of the adsorptive regions. With the blocking process, the problems are capable of being prevented from occurring in that, instead of the enzyme-labeled antibody being subjected to the specific binding with the antigen of the labeled receptor or the labeled ligand, the enzyme-labeled antibody is directly bound to the adsorptive regions of the biochemical analysis unit.

Thereafter, the reaction liquid, which contains the enzyme-labeled antibody, is forcibly caused to flow such that the reaction liquid flows across each of the adsorptive regions of the biochemical analysis unit, and the enzyme-labeled antibody is thus subjected to the specific binding with the labeled receptor or the labeled ligand. The enzyme-labeled antibody is the antibody with respect to the labeling substance of the labeled receptor or the labeled ligand, which antibody has been labeled with an enzyme. (In cases where the labeling substance of the labeled receptor or the labeled ligand is an antibody, the enzyme-labeled antibody is the antigen with respect to the labeling substance of the labeled receptor or the labeled ligand, which antigen has been labeled with an enzyme.)

After the enzyme-labeled antibody has thus been subjected to the specific binding with the labeled receptor or the labeled ligand, the biochemical analysis unit is taken out from the reactor. Thereafter, a chemical luminescence substrate is brought into contact with the enzyme-labeled antibody, which has been specifically bound to the labeled receptor or the labeled ligand. Specifically, the chemical luminescence substrate is brought into contact with the enzyme-labeled antibody from the side of the biochemical analysis unit, which side is opposite to the side provided with the signal absorbing layer.

In cases where the chemical luminescence substrate and the enzyme are brought into contact with each other, the chemical luminescence having wavelengths falling within the visible light wavelength range is produced. Therefore, the produced chemical luminescence may be detected photoelectrically from the side of the biochemical analysis unit, which side is opposite to the side provided with the signal absorbing layer. Since the enzyme-labeled antibody is ordinarily soft and sticky, it often occurs that the enzyme-labeled antibody remains unremoved with the ordinary washing operation described above. However, during the flowing of the reaction liquidwithin the reactor, the enzyme-labeled antibody clings substantially to the signal absorbing layer side of the biochemical analysis unit. Therefore, the chemical luminescence may be detected from the side of the biochemical analysis unit, which side is opposite to the side provided with the signal absorbing layer. In such cases, the chemical luminescence, which is produced when the linkage of the chemical luminescence substrate is broken by the enzyme of the enzyme-labeled antibody that clings to the signal absorbing layer instead of being specifically bound to the labeled receptor or the labeled ligand, is capable of being absorbed by the signal absorbing layer, and the image data for a biochemical analysis free from any noise signal is capable of being formed.

The present invention will further be illustrated by the following examples.

### Examples

### [Preparation of nylon liquid 1]

After 13. 7g of 6, 6-nylon was dissolved in 71.3g of formic acid, the resulting solution was allowed to stand overnight. Thereafter, 15g of water was added to the solution, and the resulting mixture was subjected to a dispersing process (1,000rpm x 60 minutes) with a homogenizer. In this manner, a nylon liquid 1 was prepared.

### [Preparation of nylon liquid 2]

After 13. 7g of 6, 6-nylon was dissolved in 71.3g of formic acid, the resulting solution was allowed to stand overnight. Thereafter, 15g of an aqueous 2% solution of a compound 1 shown below was added to the solution, and the resulting mixture was subjected to a dispersing process (1, 000rpm × 60 minutes) with a homogenizer. In this manner, a nylon liquid 2 was prepared.

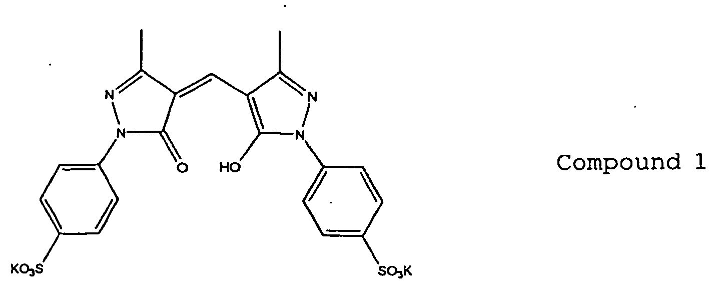

### [Preparation of nylon liquid 3]

After 13.7g of 6, 6-nylon was dissolved in 71.3g of formic acid, the resulting solution was allowed to stand overnight. Thereafter, 15g of an aqueous 2% solution of. a compound 2 shown below was added to the solution, and the resulting mixture was subj ected to a dispersing process (1,000rpm × 60 minutes) with a homogenizer. In this manner, a nylon liquid 3 was prepared.

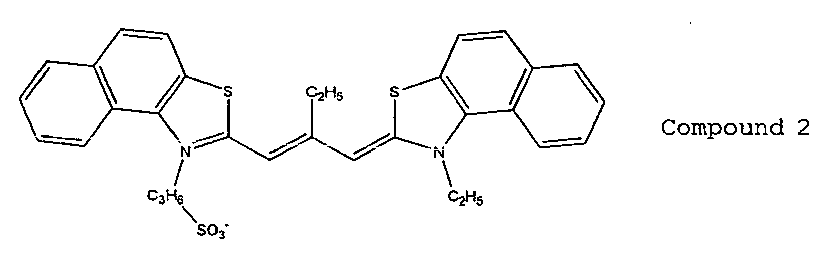

### [Preparation of nylon micro-filters]

Each of the nylon liquids 1, 2, and 3 described above was coated onto a stainless steel base plate by use of an applicator. The coating was performed under various conditions listed in Table 1 below. (As for the coating of a two-layer constitution, a second layer was overlaid upon a first layer as shown in Table 1 below.) Thereafter, the resulting coating layer was dipped in an aqueous 45% formic acid solution for 10 minutes. Thereafter, the coating layer was washed with running water for 10 minutes. The coating layer was then separated from the stainless steel base plate, set on an aluminum frame, and dried. In this manner, each of nylon micro-filters was obtained.

**Table 1**

| Level | First layer | | Second layer | |
|---|---|---|---|---|
| | Kind of nylon liquid | Coating rate (ml/m²) | Kind of nylon liquid | Coating rate (ml/m²) |
| 1 | 1 | 400 | - | - |
| 2 | 1 | 200 | 1 | 200 |
| 3 | 2 | 400 | - | - |
| 4 | 2 | 200 | 2 | 200 |
| 5 | 2 | 200 | 1 | 200 |
| 6 | 3 | 200 | - | - |
| 7 | 3 | 200 | 3 | 200 |
| 8 | 3 | 200 | 1 | 200 |

### [Preparation of biochemical analysis units]

With an etching technique, 1, 600 fine holes were formed in a SUS304 sheet (acting as a base plate material sheet) having a size of 90mm × 70mm and a thickness of 100µm. Each of the fine holes had a circular opening region having a hole diameter of 0.3mm. The fine holes were formed at a hole pitch of 0.4mm and a hole spacing of 0.1mm.

Thereafter, an adhesive agent was applied to one surface of the base plate material sheet, and the adhesive agent, which entered into the holes having been formed in the base plate material sheet, was removed by suction. The adhesive agent remaining on the surface of the base plate material sheet was then dried. Thereafter, one of the nylon micro-filters prepared in the manner described above was superposed upon the surface of the base plate material sheet, which surface had been coated with the adhesive agent. (As for the nylonmicro-filter having the two-layer constitution, the nylon micro-filter was superposed upon the surface of the base plate material sheet, such that the second layer of the nylon micro-filter came into contact with the base plate material sheet.) The combination of the nylon micro-filter and the base plate material sheet was then heated to a temperature of 150°C and pressed under pressure such that the pressure per 1cm² was 300kg. The nylon micro-filter was thus press-fitted into the fine holes of thebaseplatematerial sheet. Inthismanner, several kinds of the biochemical analysis units, each of which comprised the base plate and the plurality of the adsorptive regions formed in the fine holes of the base plate, were prepared. Thereafter, ultraviolet light having a wavelength of 2 53.7nm was irradiated at a rate of at least 1mJ/cm to each of the biochemical analysis units having been prepared. and each of the biochemical analysis units was thus sterilized with the ultraviolet light.

The compound 1 contained in the nylon liquid 2 described above is a dye capable of absorbing the chemical luminescence (having a wavelength of approximately 475nm) produced by CDP-star, which is a chemical luminescence substrate. The compound 2 contained in the nylon liquid 3 described above is a dye capable of absorbing the fluorescence (having a wavelength of approximately 570nm) producedby a f luoro chrome Cy3. With each of the biochemical analysis units having been prepared by use of one of the nylon micro-filters of levels 3, 4, 5, 6, 7, and 8 listed in Table 1, which nylon micro-filter contains the dye or the fluoro chrome described above, the noise signal was suppressed to be lower than with each of the biochemical analysis units having been prepared by use of one of the nylon micro-filters of levels 1 and 2 listed in Table 1, which nylon micro-filter does not contain the dye or the fluoro chrome described above. It is considered that the aforesaid effects of each of the biochemical analysis units having been preparedby use of one of the nylon micro-filters of levels 3, 4, 5, 6, 7, and 8 listed in Table 1, which nylon micro-filter contains the dye or the fluoro chrome described above, are obtained since the dye or the fluoro chrome described above absorbs the noise signal, which should not be detected, and the signal, which passes through the adsorptive material that is connected at the one surface of the base plate. and which thus propagates from a certain hole of the base plate to an adjacent hole of the base plate.

As described above, with the biochemical analysis unit in accordance with the present invention, the signal absorbing layer is capable of absorbing the noise signal, which comes from the labeled receptor or the labeled ligand having been agglomerated or the enzyme-labeled antibody having clogged the porous adsorptive regions of the biochemical analysis unit, and which should not be detected, and the signal, which passes through the adsorptive material that is connected at the one surface of the base plate. and which thus propagates from a certain hole of the base plate to an adjacent hole of the base plate. Therefore, the signal coming from the labeling substance is capable of being detected accurately, and accurate data for a biochemical analysis is capable of being obtained.

## Claims

1. A biochemical analysis unit (1), comprising:
i) a base plate (2), which has a plurality of holes (3), and
ii) a porous adsorptive material, which is filled in each of the plurality of the holes (3) of the base plate (2) and forms each of a plurality of adsorptive regions (4),
the porous adsorptive material, which constitutes each of the adsorptive regions (4), being connected with the porous adsorptive material, which constitutes an adjacent adsorptive region (6), at one of surfaces of the base plate (2),
**characterized in that** the biochemical analysis unit further comprises a signal absorbing layer (5) for absorbing a signal, which passes through the porous adsorptive material that is connected at the one surface of the base plate, and which thus propagates from a certain hole (3a) of the base plate toward an adjacent hole (3b) of the base plate (2), said signal absorbing layer (15) being located opposite to the side, where sigal detecting is performed.

2. A biochemical analysis unit as defined in Claim 1 wherein the signal absorbing layer (5) is formed at only an area of a continuous region of the porous adsorptive material that is connected at the one surface of the base plate (2), which area (6) is located just under the base plate (2) and is other than the areas of the adsorptive regions formed in the holes of the base plate.

3. A biochemical analysis unit as defined in Claim 1 wherein the signal absorbing layer (5) is formed at only an area, which is located just under each of the adsorptive regions formed in the holes (3) of the base plate (2).

4. A biochemical analysis unit as defined in Claim 1 wherein the signal absorbing layer is formed over an entire area of a continuous region of the porous adsorptive material that is connected at the one surface of the base plate.

5. A biochemical analysis unit as defined in any of Claims 1 to 4 wherein the base plate is constituted ofa materialhaving radiation attenuating properties and/or light attenuating properties.

6. A biochemical analysis unit (1), comprising:
i) a base plate (12), which has a plurality of holes (13), each of the plurality of holes being a through-hole and
ii) a porous adsorptive material, which is filled in each of the plurality of the holes (13) of the base plate and forms each of a plurality of adsorptive regions (14),
**characterized in that** each of the adsorptive regions (14) is provided with a signal absorbing layer (15) for absorbing a noise signal, which will otherwise be detected from the adsorptive region, said signal absorbing layer (15) being located opposite to be side, where signal detecting is performed.

7. A biochemical analysis unit as defined in Claim 6 wherein the base plate (12) is constituted of a material having radiation attenuating properties and/or light attenuating properties.

## Patentansprüche

1. Biochemische Anlayseeinheit (1) aufweisend:
i) Eine Grundplatte (2), die eine Mehrzahl von Löchern (3) hat, und
ii) ein poröses adsorptionsfähiges Material, das in jedes der Mehrzahl der Löcher (3) der Grundplatte (2) gefüllt ist und jeden von einer Mehrzahl von adsorptionsfähigen Bereichen (4) bildet,
wobei das poröse adsorptionsfähige Material, das jeden der adsorptionsfähigen Bereiche (4) ausmacht, mit dem porösen adsorptionsfähigen Material, das einen benachbarten adsorptionsfähigen Bereich (6) ausmacht, an einer der Oberflächen der Grundplatte (2) verbunden ist,
**dadurch gekennzeichnet, dass** die biochemische Analyseeinheit außerdem eine signalabsorbierende Schicht (5) zum Absorbieren eines Signals, das durch das poröse, adsorptionsfähige, an der einen Oberfläche der Grundplatte verbundene Material hindurch geht, und das sich so von einem bestimmten Loch (3a) der Grundplatte in Richtung auf ein benachbartes Loch (3b) der Grundplatte (2) ausbreitet, aufweist, wobei die signalabsorbierende Schicht (5) entgegengesetzt zu der Seite, an der der Signalnachweis durchgeführt wird, angeordnet ist.

2. Biochemische Analyseeinheit wie in Anspruch 1 definiert, bei der die signalabsorbierende Schicht (5) nur in einer Zone eines kontinuierlichen Bereichs des porösen adsorptionsfähigen Materials, das an einer Oberfläche der Grundplatte (2) verbunden ist, ausgebildet ist, wobei sich die Zone (6) gerade unter der Grundplatte (2) befindet und verschieden ist von den Zonen der adsorptionsfähigen Bereiche, die in den Löchern der Grundplatte ausgebildet sind.

3. Biochemische Analyseeinheit wie in Anspruch 1 definiert, bei der die signalabsorbierende Schicht (5) nur in einer Zone ausgebildet ist, die sich gerade unter jedem der adsorptionsfähigen Bereiche, die in den Löchern (3) der Grundplatte (2) ausgebildet sind, befindet.

4. Biochemische Analyseeinheit wie in Anspruch 1 definiert, bei der die signalabsorbierende Schicht über die gesamte Zone eines kontinuierlichen Bereichs des porösen adsorptionsfähigen Materials, das an der einen Oberfläche der Grundplatte verbunden ist, ausgebildet ist.

5. Biochemische Analyseeinheit wie in einem der Ansprüche 1 bis 4 definiert, bei der die Grundplatte aus einem Material mit strahlungsabschwächenden Eigenschaften und/oder lichtabschwächenden Eigenschaften ausgebildet ist.

6. Biochemische Analyseeinheit (1) aufweisend:
i) eine Grundplatte (12), die eine Mehrzahl von Löchern (13) hat, wobei jedes der Mehrzahl von Löchern ein durchgehendes Loch ist, und
ii) ein poröses adsorptionsfähiges Material, das in jedes der Mehrzahl von Löchern (13) der Grundplatte gefüllt ist und jeden von einer Mehrzahl von adsorptionsfähigen Bereichen (14) bildet,
**dadurch gekennzeichnet, dass** jeder der adsorptionsfähigen Bereiche (14) mit einer signalabsorbierenden Schicht (15) zum Absorbieren eines Rauschsignals, das ansonsten von dem adsorptionsfähigen Bereich erfasst wird, ausgestattet ist, wobei sich die signalabsorbierende Schicht (15) entgegengesetzt zu der Seite, an der die Signalerfassung durchgeführt wird, befindet.

7. Biochemische Analyseeinheit wie in Anspruch 6 definiert, bei der die Grundplatte (12) von einem Material gebildet wird, das strahlungsabschwächende Eigenschaften und/oder lichtabschwächende Eigenschaften hat.

## Revendications

1. Unité d'analyse biochimique (1), comprenant :
i) une plaque de base (2) qui comporte une pluralité de trous (3) et
ii) un matériau adsorbant poreux qui remplit chacun de la pluralité de trous (3) de la plaque de base (2) et forme chacune d'une pluralité de régions adsorbantes (4),
le matériau adsorbant poreux qui constitue chacune des régions adsorbantes (4) étant relié avec le matériau adsorbant poreux qui constitue une région adsorbante (6) adjacente, à une des surfaces de la plaque de base (2),
**caractérisée en ce que** l'unité d'analyse biochimique comprend en outre une couche d'absorption de signal (5) destinée à absorber un signal, lequel traverse le matériau adsorbant poreux relié à une des surfaces de la plaque de base et se propage ensuite d'un certain trou (3a) de la plaque de base à un trou adjacent (3b) de la plaque de base (2), ladite couche d'absorption de signal (5) étant située à l'opposé du côté où la détection du signal est réalisée.

2. Unité d'analyse biochimique selon la revendication 1, où la couche d'absorption de signal (5) est formée sur uniquement une zone d'une région continue du matériau adsorbant poreux relié à une des surfaces de la plaque de base (2), laquelle zone (6) est située juste en-dessous de la plaque de base (2) et est différente des zones des régions adsorbantes formées dans les trous de la plaque de base.

3. Unité d'analyse biochimique selon la revendication 1, où la couche d'absorption de signal (5) est formée sur uniquement une zone située juste en-dessous de chacune des régions adsorbantes formées dans les trous (3) de la plaque de base (2).

4. Unité d'analyse biochimique selon la revendication 1, où la couche d'absorption de signal est formée au-dessus d'une zone entière d'une région continue du matériau adsorbant poreux relié à une surface de la plaque de base.

5. Unité d'analyse biochimique selon l'une quelconque des revendications 1 à 4, où la plaque de base est constituée d'un matériau ayant des propriétés d'atténuation des radiations et/ou d'atténuation de la lumière.

6. Unité d'analyse biochimique (1), comprenant :
i) une plaque de base (12) qui comporte une pluralité de trous (13), chacun de la pluralité de trous étant un trou traversant, et
ii) un matériau adsorbant poreux, qui remplit chacun de la pluralité de trous (13) de la plaque de base et forme chacune d'une pluralité de régions adsorbantes (14),
**caractérisée en ce que** chacune des régions adsorbantes (14) est dotée d'une couche d'absorption de signal (15) destinée à absorber un signal de bruit qui serait sinon détecté par la région adsorbante, ladite couche d'absorption de signal (15) étant située à l'opposé du côté où la détection du signal est réalisée.

7. Unité d'analyse biochimique selon la revendication 6, où la plaque de base (12) est constituée d'un matériau ayant des propriétés d'atténuation des radiations et/ou d'atténuation de la lumière.
